# EUROPEAN PATENT APPLICATION

(11) **EP 0 595 568 A1**
(43) Date of publication of application: **04.05.1994**
(21) Application number: 93308466.7
(22) Date of filing: 25.10.1993
(51) Int. Cl.: G01N 3/46

(54) **Portable means and method for comparative hardness test**

(30) Priority: 26.10.1992 IL 10355492
(71) Applicant: NOGA ENGINEERING LTD., Nahariya (IL)
(72) Inventor: Hirsch, Mordechai, Naharyia (IL)
(74) Representative: Cropp, John Anthony David

(57) **Abstract**

The invention provides a method and means for the comparative measurement of hardness of metal parts. The means of the invention comprises a set of metal pins (2) each being of a different hardness, while the method of the invention comprises scratching a metal part (8) consecutively by metal pins (2) of decreasing hardness until a pin is reached which does not scratch into the metal part but slips, indicating the hardness of the metal part ranges between the hardness of said slipping pin and the hardness of the softest scratching pin.

## Description

### FIELD OF THE INVENTION

The invention relates in general to a method and means for measuring the hardness of materials. More particularly the invention relates to the comparative measurement of hardness of metals by simple portable means.

### BACKGROUND OF THE INVENTION

In the metalworking trade, steel parts of varying hardness are in common use. Frequently it is important to know the exact hardness of a certain part.

There are many well-known instruments for testing the hardness of materials. For example, one of the most widely-used test utilizes the Rockwell system under which the hardness of metals is determined by indenting the metal part to be tested with a diamond cone under a specified load and measuring the depth of penetration. Other well-known tests are Brinell hardness test which measures the hardness of a material by measuring the diameter of indentation produced by a hard steel or tungsten carbide ball under standard conditions of loading, and the Vickers test which utilizes a diamond pyramid for the measurement. However, all available instruments are elaborate machines which are very costly. A further disadvantage of those known hardness tests is the fact that the part to be tested has to be brought to the machine, and that the machine can test parts of limited size only. Thus, the testing of hardness of large metal parts cannot be carried out directly. In order to overcome the latter disadvantage some portable hardness testers have been suggested, but their use is very limited due to their high cost and complexity.

A simple method for obtaining an estimated value of hardness, which is sometimes utilized in the tool industry, is the use of a metal working file. Thus a skilled toolmaker can estimate the hardness of a steel part by carefully trying to file it, feeling how well the file "bites" into the metal.

Recently, complete sets of files, each one a little harder than its predecessor, have become available. Each file is marked with its actual hardness. A file cannot "bite" into a part which is harder than the file itself. The user of this set can start by filing the part to be tested with the hardest file, moving to the less harder one, until he reaches the stage when the file does not "bite" into the metal, but slips. He can then establish the approximate hardness of the part.

A major disadvantage of the file method is that files lose their keen cutting edge after some use and then, when a file does not cut but slips, one might come to the erroneous conclusion that the tested part is harder than it is in reality. Blunt files, therefore, become useless and must be replaced quite frequently by new files.

### BRIEF DESCRIPTION OF THE INVENTION

The object of the present invention is to provide a reliable, inexpensive method and means for testing the hardness of materials.

It is a further object of the invention to provide portable means for the measurement of hardness.

It is yet a further object of the invention to provide long-lasting means for measuring hardness, that will not wear out frequently.

In accordance with the invention there is provided a method for comparative measurement of hardness of a metal part utilizing a set of pins, each being of different hardness comprising scratching said part consecutively by metal pins of decreasing hardness, until a pin is reached which does not scratch into the metal part but slips, indicating the hardness of the metal part ranges between the hardness of said non-scratching pin and the hardness of the softest scratching pin.

The method of the invention is preferably applicable to the measurement of hardness of steels being graded from about 20 for very soft steel up to 68 for very hard steel on the Rockwell C scale.

The invention also provides for a set of pins of various hardness adapted for testing the hardness of metal parts. The pins may be set in a holder to be comfortably held in the hand of the user.

Each pin may be set in an individual holder or alternatively several pins may be held in one holder.

It is preferable in accordance with the invention that the ends of the pins be ground flat and the sharp circumferential edge of the pin is used for scratching the part to be measured. Thus, in accordance with the invention when the edge of the pin becomes blunt the end of the pin is further ground flat by any known grinding means to allow for further testing with the same pin.

In accordance with another embodiment of the invention an extension of the pin may be within the holder of the pin, thus after several grindings of the end of the pin, when the pin becomes too short, it may be extended from the holder.

### DETAILED DESCRIPTION OF THE INVENTION

The invention is illustrated in more detail by way of example only, with reference to the accompanying drawings in which:
Figure 1 is a side view of a pin according to the invention when placed in relation to a metal surface the hardness of which is to be tested;
Figure 2 is an enlarged view of the pin shown in figure 1, having sharp edges;
Figure 3 is an enlarged view of the pin shown in figure 2 having blunt edges;
Figure 4 is a side view of a handle holding 4 pins, varying in their hardness.

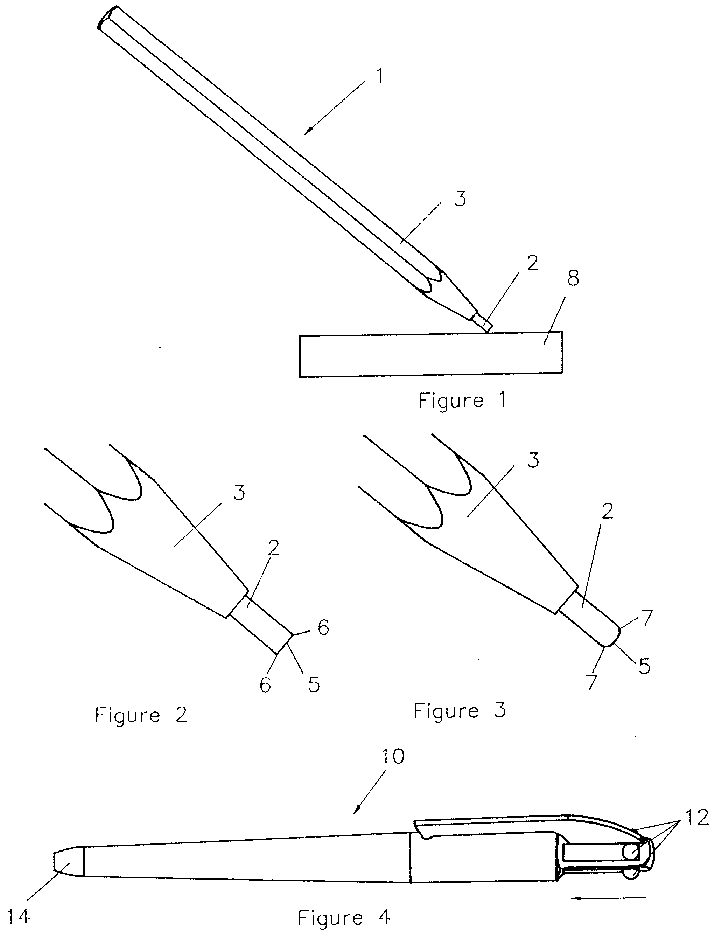

Device 1 shown in Figure 1 comprises a steel pin 2 set in holder 3. Device 1 is similar to a pencil and may be gripped by the user in a similar way to gripping a pencil for writing. The hardened steel pin 2 which replaces the lead rod in a pencil has a ground flat end 5 with a sharp circumferential edge 6 as clearly shown in Figure 2.

Device 1 is presented to a metal part 8, the hardness of which is to be tested, like a pencil to a sheet of paper for writing. With the application of some pressure a short scratch is scribed by pin 2 into the surface of part 8. Using a set of pins of varying hardness, starting with a hard pin and thereafter using pins of decreasing hardness, a point will be reached, that the pin does not scratch into the metal, but slips. Then, actual hardness of the tested part can be established; subject to the pins being conspicuously marked with their respective hardnesses.

The pins may be marked by any suitable means such as engraving, printing, coloring in different color, or by any available means of marking to distinguish between the various pins of various hardnesses.

After many uses of the pin along the circumference of sharp edge 6, the edge becomes blunt as shown in Figure 3 under nunierical value 7 and is no longer adapted for scratching parts to be tested. The bluntness of the pin is visible to the user since the blunt part of the steel pin becomes shiny; therefore the user knows that the time has come to restore the sharp edge of the pin by grinding, thereby slightly shortening the pin.

The regrinding of each pin can be done by carefully holding it against a rotating grinding wheel. However, in order to enhance loiiger life for each pin, it is actually sufficient to place a hand hone on a table and grind the pin by holding it vertically against the hone with slight pressure, and making irregular back and forth motions, until the bluntness has disappeared.

The hardness of each pin depends upon the number of pins which make up a set. If, for instance, a particular set consists of four pins, it could be niade in the following Rockwell C hardnesses: 20, 35, 50, and 65. To make up a set of say, 8 pins, the total range of hardness could be increased, so the softest pin could be made in Rockwell C 15, and the hardest in Rockwell C 68.

The diameter in which each pin is made, is not of great importance. However, the smaller the diameter, the easier it is to penetrate into the metal, but too small a diameter will create the danger of breakage. It is therefore preferable to have the pins with a diameter ranging between 1 and 3mm.

Holder 10 shown in Figure 4 is a known 4 color ball point pen which may be adapted to hold four steel pins of various hardness instead of four ball point pens. Thus, by pushing one of the four projections 12 (only three of which are shown in the drawing) in the direction of the arrow against spring pressure the projection will snap into an internal catch and the desired pin will protrude out of end 14. When a pin of another hardness is desired, the respective projection must be moved forward. This forward motion at first releases the previous pin from the catch, so that it jumps back to its "home" position, and then the new pin will be locked in the protruded position ready for scratching a part to be tested.

Figure 4 shows one design which can be utilized to hold a number of hardness "scratch testing" pins in one pencil-like pocket tool. Other mechanisms, allowing a larger number of pins, are feasible.
Alternative pocket-size hardness testers may be produced by adopting the system used in many "multiple-blade" screwdrivers, such as a handle having a collet and a collet closing nut at its front end. The other end of the handle has a recess in which all the pins are held. Any of the pins can be removed from the back compartment and clamped in the collet in order to perform the scratch test.

Another alternative hardness tester is a kit having a set of single "pencils" as shown in Figure 1, wherein each "pencil" is marked with its respective hardness and all "pencils" are packed in a portable box.

## Claims

1. A method for comparative measurement of hardness of a metal part (8) utilizing a set of pins (2), each being of different hardness, comprising scratching said part consecutively by metal pins of decreasing hardness, until a pin is reached which does not scratch into the metal part but slips, indicating the hardness of the metal part ranges between the hardness of said slipping pin and the hardness of the softest scratching pin.

2. The method according to claim 1 wherein the metal part is made of steel.

3. A set of pins each being of different hardness adapted for testing the hardness of a metal part by scratching said part consecutively by metal pins of decreasing hardness, until a pin is reached which does not scratch into the metal part but slips, indicating the hardness of the metal part ranges between the hardness of said slipping pin and the hardness of the softest scratching pin.

4. A set of pins according to claim 3 wherein each pin is set in an individual handle (3).

5. A set of pins according to claim 3 wherein the set of pins is set in one handle (10).

6. A set of steel pins according to claim 3 having hardness ranging between Rockwell C 15 and C 68.

7. A steel pin from a set of pins according to claim 3 having a diameter ranging between 1 to 3 millimeter.

8. A steel pin from a set of pins according to claim 3, wherein the end (5) of the pin is ground flat and the sharp circumferential edge (6) of said end is used for scratching the steel part (8) to be tested.

9. A steel pin from a set of pins according to claim 3 wherein said pin is extendable from the handle (3) (10).
